(19) Europäisches Patentamt
European Patent Office
Office européen des brevets

(11) Publication number: **0 051 279**
**B1**

(12) # EUROPEAN PATENT SPECIFICATION

(45) Date of publication of patent specification: **04.09.85**

(21) Application number: **81109243.6**

(22) Date of filing: **29.10.81**

(51) Int. Cl.[4]: **C 07 H 15/24,** A 61 K 31/70, C 07 H 13/04 // C07H15/04, C07D309/30

(54) **Anthracycline glycosides, intermediate compounds, process for preparing both and pharmaceutical compositions.**

(30) Priority: **01.11.80 GB 8035195**

(43) Date of publication of application: **12.05.82 Bulletin 82/19**

(45) Publication of the grant of the patent: **04.09.85 Bulletin 85/36**

(84) Designated Contracting States: **AT BE CH DE FR GB IT LI LU NL SE**

(56) References cited:
**EP-A-0 014 425**
**EP-A-0 024 727**
**FR-A-2 264 555**
**FR-A-2 269 536**
**GB-A-2 034 707**

(73) Proprietor: **FARMITALIA CARLO ERBA S.p.A.**
**Via Carlo Imbonati n.24**
**I-20159 Milan (IT)**

(72) Inventor: **Bargiotti, Alberto**
**Via Donati n.8**
**Milan (IT)**
Inventor: **Cassinelli, Giuseppe**
**Via G. Matteotti n. 13**
**Voghera (Pavia) (IT)**
Inventor: **Penco, Sergio**
**Via Crimea n.13**
**Milan (IT)**
Inventor: **Arcamone, Federico**
**Via 4 Novembre, 26**
**Nerviano (Milan) (IT)**
Inventor: **Casazza, Anna Maria**
**Via Guido Reni n. 26**
**Milan (IT)**

(74) Representative: **Hansen, Bernd, Dr.rer.nat. et al**
**Hoffmann, Eitle & Partner Patentanwälte**
**Arabellastrasse 4**
**D-8000 München 81 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 051 279 B1

**Description**

The invention relates to anthracycline glycosides, to processes for their preparation, to pharmaceutical compositions containing them and to amino-deoxy sugar derivatives used as intermediates in their preparation, as well as a process for preparing same.

The invention provides anthracycline glycosides of the general formula I and their pharmaceutically acceptable acid addition salts

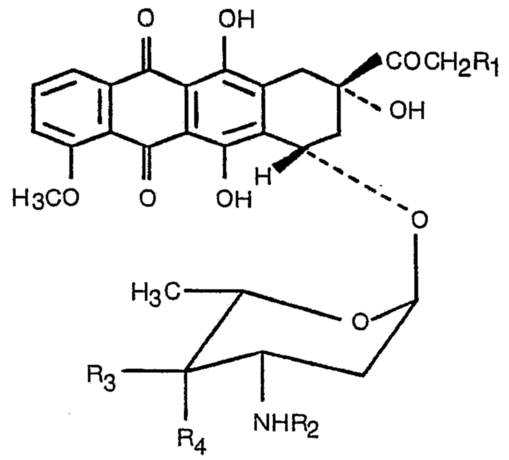

I

wherein $R_1$ represents a hydrogen atom or a hydroxy group, $R_2$ represents a hydrogen atom or an acyl group, $R_3$ represents a methyl or an aminomethyl group, and $R_4$ represents a hydrogen atom or a hydroxy group with the proviso that when $R_3$ represents a methyl group, $R_4$ does not represent a hydroxy group and when $R_3$ represents an aminomethyl group, $R_4$ does not represent a hydrogen atom; or $R_3$ and $R_4$ together represent a methylene group.

The invention further provides a process for the preparation of an anthracycline glycoside of the general formula I wherein $R_1$ represents a hydrogen atom and $R_2$, $R_3$ and $R_4$ are as herein defined, the process comprising condensing daunomycinone with
2,3,4,6-tetra-deoxy-4-C-methylene-3-trifluoroacetamido-L-threo-hexopyranosyl chloride (III—A),
2,3,4,6-tetradeoxy-4-C-methyl-3-trifluoroacetamido-L-arabino-hexopyranosyl chloride (III—B) or
2,3,6-trideoxy-4-C-trifluoroacetamidomethyl-3-trifluoroacetamido-3-O-trifluoroacetyl-L-lyxo-hexopyranoxyl chloride (III—C)

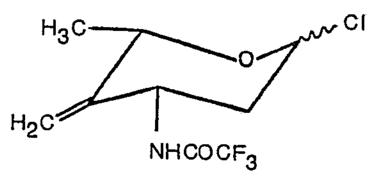

III—A

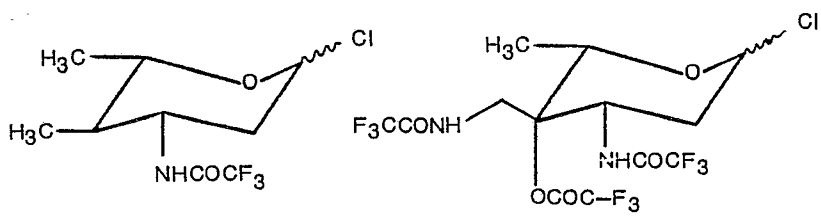

III—B III—C

to give respectively a protected glycoside IV—A (I: $R_1 = H$, $R_2 = COCF_3$, $R_3 + R_4 = CH_2$), IV—B (I: $R_1 = R_4 = H$, $R_2 = COCF_3$, $R_3 = CH_3$) or IV—C (I: $R_1 = H$, $R_2 = COCF_3$, $R_3 = CH_2—NH—COCF_3$, $R_4 = O—CO—CF_3$), and removing the protecting groups to give respectively a daunorubicin analogue *V—A* (I: $R_1 = R_2 = H$, $R_3 + R_4 = CH_2$), *V—B* (I: $R_1 = R_2 = R_4 = H$, $R_3 = CH_3$), or *V—C* (I: $R_1 = R_2 = H$, $R_3 = CH_2NH_2$, $R_4 = OH$). This may be isolated as its hydrochloride. The condensation of daunomycinone with III—A, III—B or III—C is carried out in the presence of silver trifluoromethanesulphonate as catalyst, preferably under conditions described in US—PS 4,112,076 of the applicant.

The corresponding doxorubicin analogues *VI—A* (I: $R_1 = OH$, $R_2 = H$, $R_3 + R_4 = CH_2$), *VI—B* (I: $R_1 = OH$, $R_2 = R_4 = H$, $R_3 = CH_3$) and *VI—C* ($R_1 = R_4 = OH$, $R_2 = H$, $R_3 = CH_2NH_2$) are obtained by C14-bromination followed by hydrolysis of the 14-bromo derivatives, preferably according to the method described in US—PS 4,112,076 of the applicant.

The compounds III—A, III—B and III—C are novel and are themselves included in the scope of the invention.

The inventions also provides pharmaceutical preparations comprising a compound of the general formula I as herein defined in admixture with a pharmaceutically acceptable diluent or carrier.

The starting material for the preparation of the intermediates *III—A* and *III—B* is methyl 2,3,6-trideoxy-4-C-methyl-3-trifluoroacetamido-α-L-lyxo-hexopyranoside (VII), a known compound described in British patent application No. 2,034,707 of the applicant. Treatment of this in anhydrous benzene with thionyl

chloride followed by aqueous sodium bicarbonate gives methyl 2,3,4,6-tetradeoxy-4-C-methylene-3-trifluoroacetamido-α-L-threo-hexopyranoside (VIII). Catalytic hydrogenation of compound VIII proceeds stereoselectively giving methyl 2,3,4,6-tetradeoxy-4-C-methyl-3-trifluoroacetamido-α-L-arabino-hexopyranoside (XI). Mild acid hydrolysis of compounds VIII and XI gives the corresponding hexopyranoses IX and XII which by treatment with trifluoroacetic anhydride are transformed into the corresponding 1-O-trifluoroacetates X and XIII. Treatment of these intermediates with dry hydrogen chloride in anhydrous diethyl ether affords the 1-chloroderivatives III—A and III—B respectively.

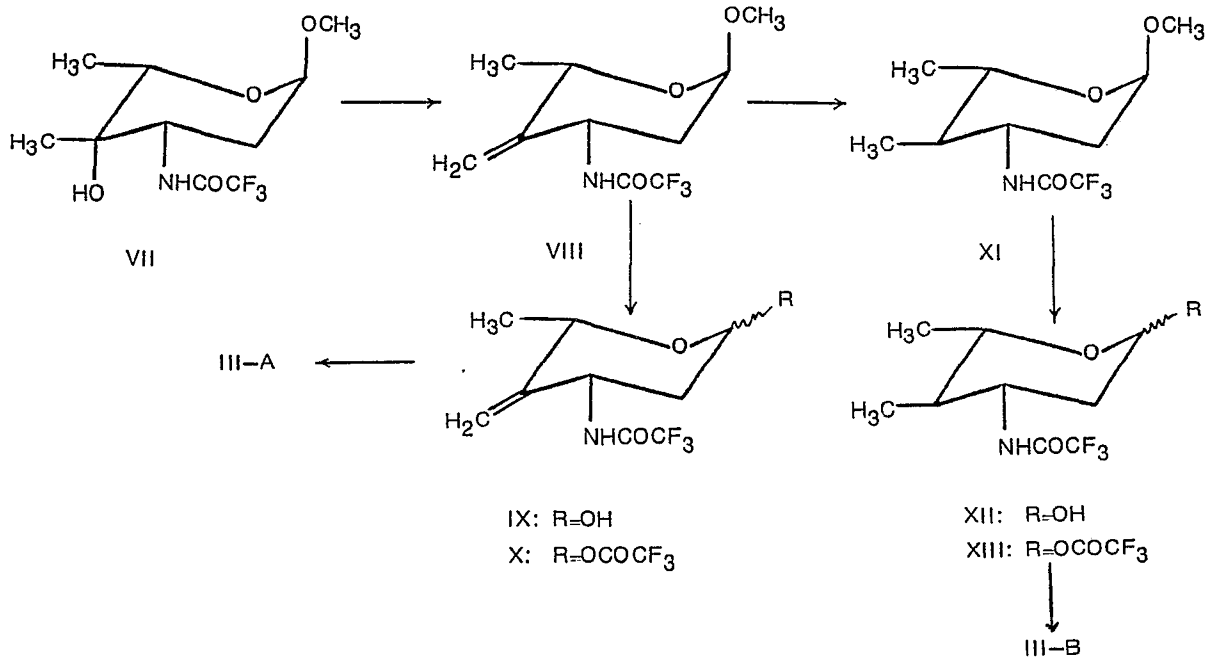

VII VIII XI

III—A

IX: R=OH
X: R=OCOCF$_3$

XII: R=OH
XIII: R=OCOCF$_3$

III—B

For the preparation of the 1-chloroderivative III—C, the key intermediate XVI is obtained by either of two different routes. Oxidation of the compound VIII with meta-chloroperbenzoic acid in 1,2-dichloroethane gives compound XVI, which may alternatively be obtained by treatment with diazomethane of methyl 2,3,6-trideoxy-3-trifluoroacetamido-α-L-threo-hexopyranosid-4-ulose (XV), a known compound described in US—PS 4,039,663 of the applicant.

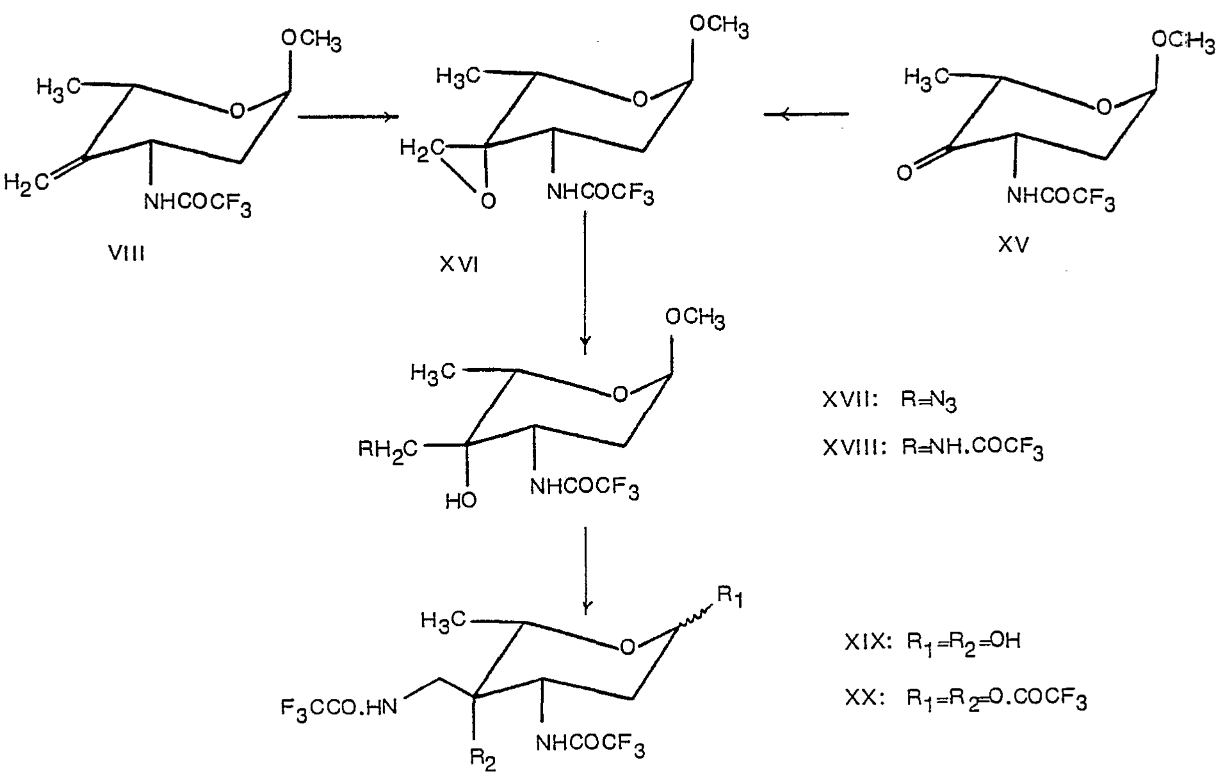

VIII XVI XV

XVII: R=N$_3$
XVIII: R=NH.COCF$_3$

XIX: $R_1$=$R_2$=OH
XX: $R_1$=$R_2$=O.COCF$_3$

Azidolysis of methyl 2,3,6-trideoxy-4-C-hydroxymethyl-4,4'-anhydro-3-trifluoroacetamido-α-L-lyxo-hexopyranoside (XVI) give the azido compound XVII. Catalytic hydrogenation followed by N-trifluoroacetylation affords compound XVIII, which on mild acid hydrolysis gives XIX. Sequential O-trifluoroacetylation and treatment with dry hydrogen chloride in anhydrous diethyl ether gives the 1-chloro-derivative III—C.

The invention is illustrated by the following examples

## Example 1

Preparation of methyl 2,3,4,6-tetradeoxy-4-C-methylene-3-trifluoroacetamido-α-L-threo-hexopyranoside (VIII)

A solution of methyl 2,3,6-trideoxy-4-C-methyl-3-trifluoroacetamido-α-L-lyxo-hexopyranoside (VII, 1 g, 3.7 mmol) in anhydrous benzene (90 ml) was treated with freshly distilled thionyl chloride (2.75 ml) and heated under reflux for 2 hours. The reaction mixture, cooled at room temperature, was washed with water and then treated under stirring with an aqueous solution of sodium hydrogen carbonate. The organic phase was separated off, washed with water and evaporated under vacuum to dryness to give the title compound (VIII, 0.82 g, 87%) as a white solid; m.p. 160—162°C, $[\alpha]_D^{20°}$ −180° (c = 1, $CHCl_3$). The PMR spectrum ($CDCl_3$) showed absorptions at 1.35 (d, $CH_3$—C-5), 3.37 (s, $CH_3O$), 4.37 (q, H—C-5), 4.83 (broad s, $CH_2$=C-4) and 4.95 δ (broad s, C-1-H).

## Example 2

Preparation of 2,3,4,6-tetradeoxy-4-C-methylene-3-trifluoroacetamido-L-threo-hexopyranosyl chloride (III—A)

A solution of the intermediate VIII (0.51 g, 2 mmol) in acetic acid (10 ml) and water (40 ml) was heated at 100°C for two hours, then evaporated to dryness to give 2,3,4,6-tetradeoxy-4-C-methylene-3-trifluoro-acetamido-L-threo-hexopyranose (IX, 0.47 g, 99%) as a white solid: m.p. 168—170°C, $[\alpha]_D^{20°}$ −160° (c = 0.5, $CHCl_3$). Treatment of IX (0.36 g, 1.5 mmol) in dry methylene dichloride (25 ml) with trifluoroacetic anhydride (4 ml) for 2 hours at 0°C and 1 hour at 20°C gave, after evaporation, the 1-O-trifluoroacetate (X), which was directly used for the preparation of the corresponding 1-chloro-derivative by treatment with dry hydrogen chloride in anhydrous diethyl ether at 0°C. After standing at 0°C overnight, the reaction mixture was evaporated to dryness to give 2,3,4,6-tetradeoxy-4-C-methylene-3-trifluoroacetamido-L-threo-hexo-pyranosyl chloride (III—A, 0.35 g, 90%), suitable for the subsequent coupling reaction without further purification.

## Example 3

Preparation of 4'-deoxy-4'-C-methylene-daunorubican (V—A)

Daunomycinone (0.76 g, 1.9 mmol) was condensed with the protected pyranosyl chloride III—A (0.34 g, 1.3 mmol) in dry methylene dichloride (90 ml) in the presence of molecular sieves (4Å-Merck, 6 g) and using silver trifluoromethanesulphonate (0.45 g, in 15 ml diethyl ether), as catalyst. After 30 minutes under vigorous stirring at room temperature, a saturated aqueous solution of sodium hydrogen carbonate was added to the reaction mixture and the organic phase was separated off, washed with water and evaporated to a small volume. It was then subjected to chromatographic purification on a silica gel column. Elution with a 97:3 by volume chloroform:acetone mixture gave pure 4'-deoxy-4'-C-methylene-N-trifluoroacetyl-daunorubican (IMI-106) (IV—A, 0.66 g, 71%): m.p. 135—136°C, $[\alpha]_D^{23°}$ +225° (c = 0.05, $CHCl_3$). The PMR spectrum ($CDCl_3$) showed absorptions at 1.42 (d, $CH_3$—C-5'), 2.39 (s, $CH_3CO$), 3.99 (s, $CH_3O$—C-4), 4.60 (q, H—C-5'), 4.95 (m, $CH_2$=C-4'), 5.17 (broad s, H—C-7), 5.52 (broad s, H—C-1'), 13.06 and 13.94 δ (two s, phenolic OHs).

A solution of IV—A (0.25 g, 0.4 mmol) in acetone (10 ml) was treated with 0.2 N aqueous sodium hydroxide (10 ml) and stirred under nitrogen at room temperature. After 3 hours the reaction mixture was adjusted to pH 3.5 with 1N aqueous hydrogen chloride and extracted with chloroform to eliminate impurities. The subsequent chloroform extraction of the aqueous phase at pH 8, concentration of the organic phase, addition of methanolic hydrogen chloride followed by precipitation with diethyl ether gave 4'-deoxy-4'-C-methylene-daunorubican (V—A) as hydrochloride (0.14 g, 61% yield): m.p. 124—126°C, $[\alpha]_D^{23°}$ +142° (c = 0.05, $CH_3OH$).

## Example 4

Preparation of 2,3,4,6-tetradeoxy-4-C-methyl-3-trifluoroacetamido-L-arabino-hexopyranosyl chloride (III—B)

Stereoselective reduction of the intermediate VIII (0.5 g, prepared as described in Example 1) in methanol (40 ml) in the presence of 20% palladium-on-charcoal (0.5 g) under pressure (20 atmospheres) gave in quantitative yield, methyl 2,3,4,6-tetradeoxy-4-C-methyl-3-trifluoroacetamido-L-arabino-hexo-pyranoside (XI) as a white solid: m.p. 132—134°C, $[\alpha]_D^{23°}$ −82° (c = 0.5, $CHCl_3$). The PMR spectrum ($CDCl_3$) showed adsorptions at: 0.90 (d, $CH_3$—C-4), 1.23 (d, $CH_3$—C-5), 1.26 (m, H—C-4), 3.42 (s, $OCH_3$), 3.70 (dq, H—C-5), 4.20 (m, H—C-3) and 4.87 δ (dd, H—C-1). A solution of XI (0.385 g, 1.5 mmol) in acetic acid (7 ml) and water (28 ml) was heated at 100°C for two hours and then evaporated to dryness to give in quantitative yield 2,3,4,6-tetradeoxy-4-C-methyl-3-trifluoroacetamido-α-L-arabino-hexopyranose (XII) as a white solid: m.p. 158—159°C, $[\alpha]_D^{23°}$ −62° (c = 0.5, $CHCl_3$).

Compound XII (0.245 g, 1 mmol) via 1-O-trifluoroacetate (XIII) was converted into the 1-chloroderivative (III—B, 0.25 g, 1 mmol) followed the same procedure as described in Example 2.

Example 5

Preparation of 4'-deoxy-4'-epi-C-methyl-daunorubicin (V—B) (XOO-0123)

Daunomycinone (0.5 g, 1 mmol) was condensed with the 1-chloroderivative (III—B, 0.26 g, 1 mmol), following the procedure described in Example 3, to give the protected glycoside 4'-deoxy-4'-epi-C-methyl-N-trifluoroacetyl-daunorubicin (IV—B, 0.41 g, 66% yield): m.p. 118—120°C with decomposition, $[\alpha]_D^{23°}$ +271° (c = 0.05, $CHCl_3$).

The PMR spectrum ($CDCl_3$) showed absorptions at: 0.97 (d, $CH_3$—C-4'), 1.33 (d, $CH_3$—C-5'), 2.47 (s, $COCH_3$), 4.10 (s, $OCH_3$—C-4), 5.28 (broad s, H—C-7), 5.53 (broad s, H—C-1'), 13.00 and 13.72 δ (two s, phenolic OHs). Removal of the protecting group, as previously described in Example 3, gave 4'-deoxy-4'-epi-C-methyl-daunorubicin (V—B, 90% yield) as its hydrochloride: m.p. 149—150°C (with decomposition), $[\alpha]_D^{23°}$ = +215° (c = 0.5, $CH_3OH$).

Example 6

Preparation of 4'-deoxy-4'-epi-C-methyl-doxorubicin (VI—B)

A solution of 4'-deoxy-4'-epi-C-methyl-daunorubicin hydrochloride (V—B, 0.53 g, 1 mmol), preparation as described in Example 5, in a mixture of anhydrous methanol (8 ml), dioxane (23 ml) and ethyl orthoformate (0.6 ml) was treated with 2.4 ml of a solution of bromine (1.86 g) in chloroform (20 ml). After 2 hours at 10°C, the reaction mixture was poured into a 2:1 by volume mixture of diethyl ether:*n*-hexane (180 ml). The resultant precipitate, after being filtered off and washed with diethyl ether to remove the acidity, was dissolved in a 1:1 by volume mixture of acetone:0.25N aqueous hydrogen bromide (40 ml). After 20 hours at 30°C the reaction mixture was treated with sodium formate (0.9 g) in water (10 ml) and stirred for 48 hours at 30°C. The reaction mixture was extracted with chloroform in order to remove some lypophilic impurities. The aqueous phase, after being adjusted to pH 7.6 with aqueous sodium hydrogen carbonate, was repeatedly extracted with chloroform. The combined chloroform extracts were dried with anhydrous sodium sulfate and evaporated to a small volume. They were then treated with anhydrous methanolic hydrogen chloride (pH 3.5) and precipitated with an excess of diethyl ether to give the title compound (VI—B) as its hydrochloride.

Example 7

Preparation of 2,3,6-trideoxy-4-C-trifluoroacetamido-methyl-3-trifluoroacetamido-4-O-trifluoroacetyl-L-lyxo-hexopyranosyl chloride (III—C)

A solution of the intermediate VIII (0.51 g, 2 mmol) and *m*-chloroperoxybenzoic acid (0.69 g, 4 mmol) in dry 1,2-dichlorethane (20 ml) was refluxed for two days. The reaction mixture was washed with aqueous sodium hydrogen carbonate and then with water and the organic layer was evaporated to give crystalline methyl 2,3,6-trideoxy-4-C-hydroxymethyl-4,4'-anhydro-3-trifluoroacetamido-L-lyxo-hexopyranoside (XVI, 0.5 g, 92% yield). The PMR spectrum ($CDCl_3$) showed adsorptions at 1.07 (d, $CH_3$—C-5), 2.69 (dd, $OCH_2$—C-4), 3.42 (s, $OCH_3$), 4.40 (q, H—C-5) and 4.85 δ (broad s, H—C-1).

Compound XVI was also prepared starting from methyl 2,3,6-trideoxy-3-trifluoroacetamido-α-L-threo-hexopyranosid-4-ulose (XV, 0.51 g, 2 mmol) in methanol (10 ml) by treatment with a solution of diazomethane in methylene dichloride (0.64 g in 20 ml). The reaction mixture was stirred at room temperature overnight and then evaporated to dryness. The crystalline residue (0.5 g, 92% yield) was identical with that obtained by the previously described route.

A solution of compound XVI (0.54 g, 2 mmol) in dioxan (15 ml) and water (5 ml) was treated with sodium azide (0.95 g) and ammonium chloride (0.425 g), and then stirred under reflux for 4 hours. The organic layer obtained after addition of chloroform and water was evaporated to dryness to give the crystalline azido derivative XVII (0.62 g in quantitative yield): m.p. 150—151°C, $[\alpha]_D^{23°}$ −179° (c = 1, $CHCl_3$). The I.R. spectrum (film) showed adsorptions at 3,380 (OH) and 2,210 ($N_3$) $cm^{-1}$. The PMR spectrum ($CDCl_3$) showed adsorptions at: 1.23 (d, $CH_3$—C-5), 3.35 (s, $CH_2$—C-4), 3.39 (s, $OCH_3$), 4.08 (q, H—C-5) and 4.76 δ (broad s, H—C-1).

Compound XVII (0.47 g, 1.5 mmol) in methanol (20 ml) was hydrogenated in the presence of 20% palladium-on-charcoal (0.5 g) under pressure (10 atmospheres). After removal of the catalyst by filtration, the solution was evaporated to a colourless syrup which was dissolved in anhydrous methylene dichloride (15 ml) and treated with trifluoroacetic anhydride (2 ml) at 0°C. After 30 minutes the reaction mixture was evaporated to dryness to give methyl 2,3,6-trideoxy-4-C-trilfluoroacetamido-methyl-3-trifluoroacetamido-α-L-lyxo-hexopyranoside (XVIII, 0.47 g, 82%): m.p. 165—166°C $[\alpha]_D^{23°}$ −59°C (c = 1, $CHCl_3$). The PMR spectrum ($CDCl_3$) showed adsorptions at 1.32 (d, $CH_3$—C-5), 3.36 (s, $OCH_3$), 3.88 (q, H—C-5) and 4.73 δ (dd, H—C-1).

A solution of XVIII (0.38 g, 1 mmol) in acetic acid (8 ml) and water (32 ml) was heated at 100°C for 3 hours, then evaporated to dryness to give compound XIX as a syrup, which was treated with trifluoroacetic anhydride (3.5 ml) in methylene dichloride (20 ml) in the presence of dimethylaminopyridine (0.035 g) for 16 hours at 10°C to give the corresponding di-O-trifluoroacetate XX. Subsequent treatment with dry hydrogen chloride at 0°C in diethyl ether gave the title compound III—C, which was used for the next step without further purification.

Example 8

Preparation of 4'-C-aminomethyl-daunorubicin (V—C) (XOO—0121)

Daunomycinone (0.48 g, 1.2 mmol) was condensed with the protected pyranosyl chloride III—C (0.48 g, 1 mmol) in anhydrous methylene dichloride (60 ml) in the presence of molecular sieve (4 Å Merck, 5 g) using silver trifluoromethane sulphonate (0.3 g, in 10 ml of diethyl ether) as catalyst. After 30 minutes under vigorous stirring at room temperature, the reaction mixture was treated with a saturated aqueous solution of sodium hydrogen carbonate. The separated organic phase was washed with water and then evaporated to dryness. The solid residue was dissolved in dry methanol (15 ml) and kept for 2 hours at room temperature. The crude product obtained by evaporating off the solvent was chromatographed on a silica gel column using a 9:1 by volume chloroform: acetone mixture was eluting agent to give pure 4'-C-trifluoroacetamido-methyl-N-trifluoroacetyl-daunorubicin (IV—C, 0.52 g, 70%): m.p. 125—127°C, $[\alpha]_D^{23°}$ +340° (c = 0.05, $CHCl_3$). The PMR spectrum ($CDCl_3$) showed adsorptions at: 1.41 (d, $CH_3$—C-5'), 2.37 (s, $COCH_3$), 4.08 (s, $CH_3$—O—C-4), 5.09 (broad s, H—C-7), 5.42 (broad s, H—C-1'), 13.11 and 13.90 δ (two s, phenolic OHs). Removal of the protecting groups, as previously described in Example 3, gave 4'-C-amino-methyl-daunorubicin (V—C, 71% yield) as its hydrochloride: m.p. 159—152°C, $[\alpha]_D^{20°}$ +229° (c = 0.04, methanol).

Example 9

Preparation of 4'-C-aminomethyl-doxorubicin (VI—C)

The 14-bromoderivative of compound V—C (prepared as described in Example 8) was obtained and successively hydroxylated at the 14-position according to the procedure described in Example 6 to give the title compound (VI—C) as its hydrochloride.

Biological activity

On HeLA cells cloning efficiently in vitro, compounds IMI-106 and XOO-0121 were markedly less active than daunorubicin, while XOO-0123 maintained a high cytotoxic activity (Table 1). As regards the in vivo tests, preliminary experiments have been carried on the P388 ascitic leukemia. Results are shown in Table 2. All the compounds exerted antitumour activity, among the 3 new derivatives, the most interesting is XOO-0121 which, at the dose of 200 mg/kg was more active than daunorubicin.

TABLE 1

Activity on HeLa cells cloning efficiently in vitro. Treatment for 24 hours.

| Compound | Dose (ng/ml) | % of controls | $ID_{50}$ (ng/ml) |
|---|---|---|---|
| Daunorubicin[a] | 12.6 | 36—9.7—18 | 9—8.3 |
| | 6.25 | 66—69—57 | |
| | 3.12 | 105—100—70 | |
| IMI-106[b] (IV—A) | 3200 | 7 | 1600—1300 |
| | 1600 | 63—29 | |
| | 800 | 115—116 | |
| XOO—0121 (V—C) | 1600 | 3.8 | 1000 |
| | 400 | 141 | |
| | 100 | 143 | |
| XOO-0123 (V—B) | 100 | 0 | 7.7 |
| | 25 | 4 | |
| | 6.2 | 54 | |

[a]Data of 3 experiments
[b]Data of 2 experiments.

TABLE 2

Activity against P388 ascitic leukemia. Treatment i.p. on day 1 after tumour inoculation.

| Compound | Dose (mg)kg) | T/C[a] (%) | Toxicity deaths |
|---|---|---|---|
| Daunorubicin[b] | 2.9 | 170—172—180 | 0/10—0/6—0/10 |
| | 4.4 | 170—168—185 | 1/10—0/6—0/10 |
| | 5.5 | 155—168—190 | 6/10—5/5—4/10 |
| IMI-106 (IV—a) | 100 | 110 | 0/5 |
| | 200 | 110 | 0/5 |
| | 400 | 130 | 0/3 |
| XOO-0121 (V—C) | 50 | 181 | 0/6 |
| | 100 | 172 | 0/5 |
| | 200 | 204 | 0/4 |
| XOO-0123 (V—B) | 4.4 | 130 | 0/10 |
| | 6.6 | 140 | 0/10 |
| | 10.0 | 140 | 0/9 |

[a]Median survival time of treated mice/median survival time of controls, %.
[b]Date of 3 experiments.

**Claims for the Contracting States: BE CH DE FR GB IT LI LU NL SE**

1. An anthracycline glycoside of the general formula I.

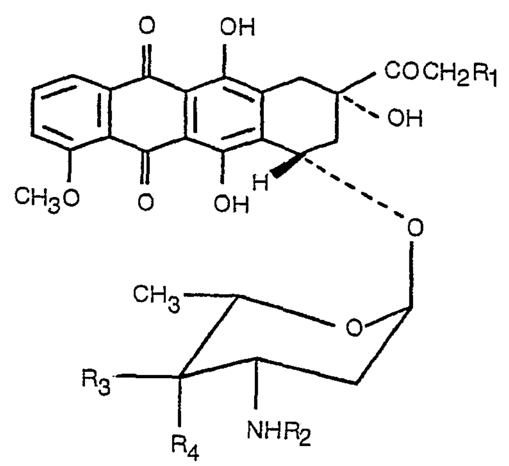

I

wherein $R_1$ represents a hydrogen atom or a hydroxy group; $R_2$ represents a hydrogen atom or an acyl group; $R_3$ represents a methyl or an aminomethyl group; $R_4$ represents a hydrogen atom or a hydroxy group with the proviso that when $R_3$ represents a methyl group, $R_4$ does not represent a hydroxy group and when $R_3$ represents an aminomethyl group, $R_4$ does not represent a hydrogen atom; $R_3$ and $R_4$ together may represent a methylene group and their pharmaceutically acceptable acid addition salts.

2. A compound according to claim 1, which is 4'-deoxy-4'-C-methylene-daunorubicin hydrochloride.
3. A compound according to claim 1, which is 4'-deoxy-4'-epi-C-methyl-daunorubicin hydrochloride.
4. A compound according to claim 1, which is 4'-deoxy-4'-epi-C-methyl-doxorubicin hydrochloride.
5. A compound according to claim 1, which is 4'-C-amino-methyl-daunorubicin hydrochloride.
6. A compound according to claim 1, which is 4'-C-amino-methyl-doxorubicin hydrochloride.

7. A process for the preparation of anthracycline glycosides according to formula I of claim 1, wherein $R_1$ represents a hydrogen atom and $R_2$, $R_3$ and $R_4$ are as defined in claim 1, characterized by condensing daunomycinone with

a) 2,3,4,6-tetra-deoxy-4-C-methylene-3-trifluoroacetamido-L-threo-hexopyranosyl chloride, or

b) 2,3,4,6-tetra-deoxy-4-C-methyl-3-trifluoroacetamido-L-arabino-hexopyranosyl chloride, or

c) 2,3,6-trideoxy-4-C-trifluoroacetamidomethyl-3-trifluoroacetamido-4-O-trifluoroacetyl-L-lyxo-hexopyranosyl chloride

to obtain respective protected glycosides, and removing the protecting groups to yield the desired daunorubicin analogues.

8. A process according to claim 7, characterized in that the daunorubicin analogues are isolated as hydrochlorides.

9. A process according to claim 7, characterized in that condensation of daunomycinone with the compounds a) to c) according to claim 7 is carried out in the presence of silver trifluoromethanesulphonate as catalyst.

10. A process for the preparation of anthracycline glycosides of formula I according to claim 1, wherein $R_1$ represents a hydroxyl group and $R_2$, $R_3$ and $R_4$ are as defined in claim 1, characterized by $C_{14}$-bromination of a compound as obtained in claims 7 to 9, followed by hydrolysis of the 14-bromo derivatives.

11. An intermediate compound for the preparation of anthracycline glycosides according to claim 1, which is 2,3,4,6-tetra-deoxy-4-C-methylene-3-trifluoroacetamido-L-threo-hexopyranosyl chloride.

12. An intermediate compound for the preparation of anthracycline glycosides according to claim 1, which is 2,3,4,6-tetra-deoxy-4-C-methyl-3-trifluoroacetamido-L-arabino-hexopyranosyl chloride.

13. An intermediate compound for the preparation of anthracycline glycosides according to claim 1, which is 2,3,6-trideoxy-4-C-trifluoroacetamidomethyl-3-trifluoroacetamido-4-O-trifluoroacetyl-L-lyxo-hexopyranosyl chloride.

14. A process for the preparation of intermediate compounds according to claims 11 and 12, characterized by

A) treatment of 2,3,6-trideoxy-4-C-methyl-3-trifluoroacetamido-α-L-lyxo-hexopyranoside in anhydrous benzene with thionyl chloride followed by aqueous sodium bicarbonate to obtain 2,3,4,6-tetradeoxy-4-C-methylene-3-trifluoroacetamido-α-L-threo-hexopyranoside;

B) catalytic hydrogenation of above-obtained compound to yield 2,3,4,6-tetradeoxy-4-C-methyl-3-trifluoroacetamido-α-L-arabino-hexopyranoside;

C) mild acid hydrolysis of compounds obtained under a) and b) to give the corresponding hexopyranoses, which by treatment with trifluoroacetic anhydride are transformed into the corresponding 1-O-trifluoroacetates;

D) treatment with dry hydrogen chloride in anhydrous diethyl ether to yield the intermediate compounds according to claims 11 and 12.

15. A process for the preparation of the intermediate compound according to claim 13, characterized by

A) oxydizing compound VIII

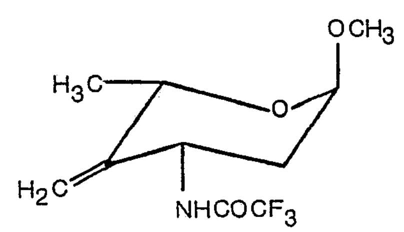

VIII

with meta-chloroperbenzoic acid in 1,2-dichloroethane, whereby compound XVI

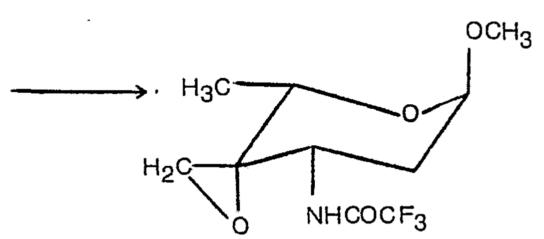

XVI

is obtained, or

B) treatment of methyl 2,3,6-trideoxy-3-trifluoroacetamido-α-L-threo-hexopyranosid 4-ulose with diazomethane, whereby above-mentioned compound XVI is obtained,

C) azidolysis of methyl-2,3,6-trideoxy-4-C-hydroxy-methyl-4,4'-anhydro-3-trifluoroacetamido-α-L-lyxo-hexopyranoside to yield the respective azido compound;

D) catalytic hydrogenation followed by N-trifluoroacetylation to yield compound XVIII

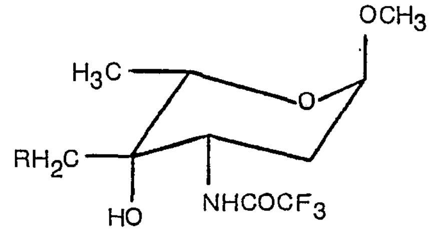

XVIII

wherein R = NH—CO—$CF_3$,

E) which on mild acid hydrolysis gives compound XIX

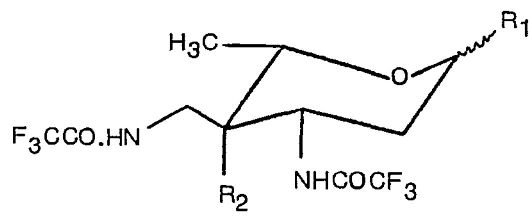

XIX

wherein $R_1 = R_2 = OH$;

F) sequential O-trifluoroacetylation and treatment with dry hydrogen chloride in anhydrous diethyl ether to give the 1-chloroderivative according to claim 13.

16. A pharmaceutical composition comprising a therapeutically effective amount of a compound according to claim 1, in combination with a carrier thereof.

**Claims for the Contracting State: AT**

1. A process for the preparation of anthracycline glycosides according to formula I

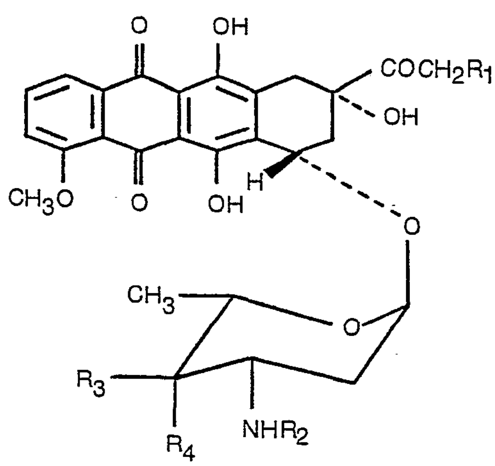

I

wherein $R_1$ represents a hydrogen atom or a hydroxy group; $R_2$ represents a hydrogen atom or an acyl group; $R_3$ represents a methyl or aminomethyl group; $R_4$ represents a hydrogen atom or a hydroxy group, with the proviso that when $R_3$ represents a methyl group, $R_4$ does not represent a hydroxy group and when $R_3$ represents an aminomethyl group, $R_4$ does not represent a hydrogenation; $R_3$ and $R_4$ may together represent a methylene group and their pharmaceutically acceptable acid addition salts, characterized in condensing daunomycinone with

(a) 2,3,4,6-tetra-deoxy-4-C-methylene-3-trifluoroacetamido-L-threo-hexopyranosyl chloride, or

(b) 2,3,4,6-tetra-deoxy-4-C-methyl-3-trifluoroacetamido-L-arabino-hexopyranosyl chloride, or

(c) 2,3,6-trideoxy-4-C-trifluoroacetamidomethyl-3-trifluoroacetamido-4-O-trifluoroacetyl-L-lyxo-hexopyranosyl chloride

to obtain respective protected glycosides, and removing the protecting groups to yield the desired daunorubicin analogues.

2. A process according to claim 1, characterized in that the daunorubicin analogues are isolated as hydrochlorides.

3. A process for the preparation of anthracycline glycosides according to claim 1, wherein $R_1$ represents a hydroxyl group and $R_2$, $R_3$ and $R_4$ are as defined in claim 1, characterized by C14-bromination of a compound as obtained in claim 1, followed by hydrolysis of the 14-bromo-derivatives.

4. A process for the preparation of intermediate compounds according to claim 1 (a) and (b), characterized by

A) treatment of 2,3,6-trideoxy-4-C-methyl-3-trifluoro-acetamido-α-L-lyxo-hexapyranoside in anhydrous benzene with thionyl chloride followed by aqueous sodium bicarbonate to obtain 2,3,4,6-tetradeoxy-4-C-methylene-3-trifluoroacetamido-α-L-threo-hexopyranoside;

B) catalytic hydrogenation of above-obtained compound to yield 2,3,4,6-tetradeoxy-4-C-methyl-3-trifluoroacetamido-α-L-arabino-hexopyranoside;

C) mild acid hydrolysis of compounds obtained under (A) and (B) to give the corresponding hexopyranoses, which by treatment with trifluoroacetic anhydride are transformed into the corresponding 1-O-trifluoroacetates;

D) treatment with dry hydrogen chloride in anhydrous diethyl ether to yield the desired intermediate compounds.

5. A process for the preparation of the intermediate compound according to claim 1 (c), characterized by

A) oxydizing compound VIII

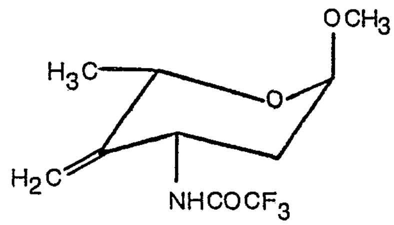

VIII

with meta-chloroperbenzoic acid in 1,2-dichloroethane, whereby compound XVI

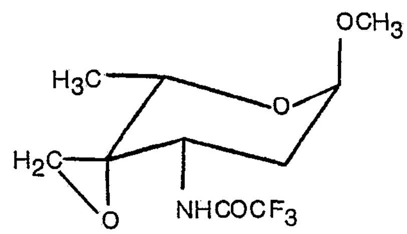

XVI

it obtained, or

B) treatment of methyl 2,3,6-trideoxy-3-trifluoroacetamido-α-L-threo-hexopyranosid-4-ulose with diazomethane, whereby above-mentioned compound XVI is obtained,

C) azidolysis of methyl-2,3,6-trideoxy-4-C-hydroxymethyl-4,4'-anhydro-3-trifluoroacetamido-α-L-lyxo-hexopyranoside to yield the respective azido compound;

D) catalytic hydrogenation followed by N-trifluoroacetylation to yield compound XVIII

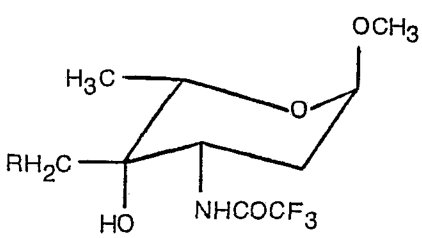

XVIII

wherein R = NH—CO—$CF_3$,

E) which on mild acid hydrolysis gives compound XIX

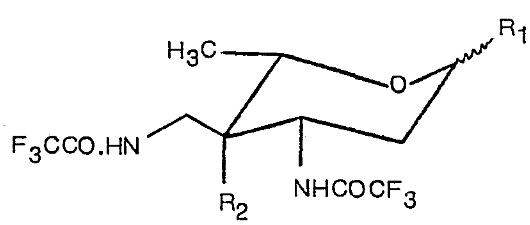

XIX

wherein $R_1 = R_2 = OH$;

F) sequential O-trifluoroacetylation and treatment with dry hydrogen chloride in anhydrous diethyl ether to give the desired 1-chloroderivative.

**Patentansprüche für die Vertragsstaaten: BE CH DE FR GB IT LI LU NL SE**

1. Anthracyclinglycosid der allgemeinen Formel I:

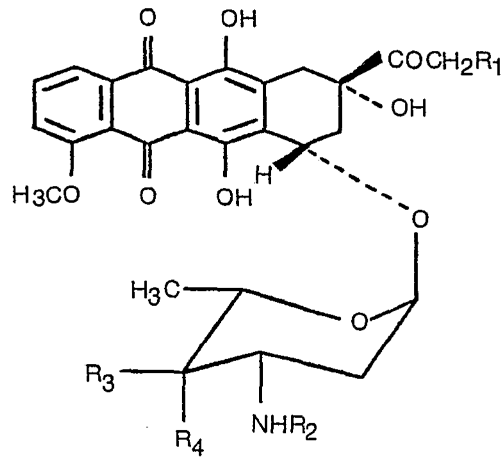

I

worin $R_1$ ein Wasserstoffatom oder eine Hydroxygruppe bedeutet; $R_2$ ein Wasserstoffatom oder eine Acylgruppe darstellt; $R_3$ eine Methyl- oder eine Aminomethylgruppe bedeutet; $R_4$ ein Wasserstoffatom oder eine Hydroxygruppe bedeutet, ausgenommen, dass, wenn $R_3$ eine Methylgruppe bedeutet, $R_4$ nicht eine Hydroxygruppe darstellt, und wenn $R_3$ eine Aminomethylgruppe bedeutet, $R_4$ nicht ein Wasserstoffatom darstellt; $R_3$ und $R_4$ zusammen eine Methylengruppe darstellen können, und deren pharmazeutisch annehmbare Säureadditionssalze.

2. 4'-Desoxy-4'-C-methylen-daunorubicin-hydrochlorid.

3. 4'-Desoxy-4'-epi-C-methyl-daunorubicin-hydrochlorid.

4. 4'-Desoxy-4'-epi-C-methyl-doxorubicin-hydrochlorid.

5. 4'-C-Aminomethyl-daunorubicin-hydrochlorid.

6. 4'-C-Aminomethyl-doxorubicin-hydrochlorid.

7. Verfahren zur Herstellung von Anthracyclinglycosiden gemäss Formel I von Anspruch 1, worin $R_1$ ein Wasserstoffatom bedeutet, und $R_2$, $R_3$ und $R_4$ die in Anspruch 1 angegebene Bedeutung haben, gekennzeichnet durch Kondensierung von Daunomycinon mit

(a) 2,3,4,6-Tetra-desoxy-4-C-methylen-3-trifluoracetamido-L-threo-hexopyranosylchlorid, oder

(b) 2,3,4,6-Tetra-desoxy-4-C-methyl-3-trifluoracetamido-L-arabino-hexopyranosylchlorid, oder

(c) 2,3,6-Tridesoxy-4-C-trifluoracetamidomethyl-3-trifluoracetamido-4-O-trifluoracetyl-L-lyxo-hexopyranosyl-chlorid,

um die jeweiligen geschützten Glycoside zu erhalten, und Entfernen der Schutzgruppen unter Erhalt der gewünschten Daunorubicinanaloge.

8. Verfahren nach Anspruch 7, dadurch gekennzeichnet, dass die Daunorubicinanalogen als Hydrochloride isoliert werden.

9. Verfahren nach Anspruch 7, dadurch gekennzeichnet, dass die Kondensation von Daunomycinon mit den Verbindungen (a) bis (c) gemäss Anspruch 7 in Gegenwart von Silber-trifluor-methansulfonat als Katalysator ausgeführt wird.

10. Verfahren zur Herstellung von Anthracyclinglycosiden der Formel I gemäss Anspruch 1, worin $R_1$ eine Hydroxylgruppe bedeutet, und $R_2$, $R_3$, und $R_4$ die in Anspruch 1 angegebene Bedeutung haben, gekennzeichnet durch C14-Bromierung einer in den Ansprüchen 7 bis 9 erhaltenen Verbindung, gefolgt von einer Hydrolyse der 14-Bromderivate.

11. Zwischenverbindung zur Herstellung von Anthracyclinglycosiden gemäss Anspruch 1, dadurch gekennzeichnet, dass diese 2,3,4,6-Tetra-desoxy-4-C-methylen-3-trifluoracetamido-L-threo-hexopyranosylchlorid darstellt.

12. Zwischenverbindung zur Herstellung von Anthracyclinglycosiden gemäss Anspruch 1, dadurch gekennzeichnet, dass diese 2,3,4,6-Tetra-desoxy-4-C-methyl-3-trifluoracetamido-L-arabino-hexopyranosylchlorid darstellt.

13. Zwischenverbindung zur Herstellung von Anthracyclinglycosiden gemäss Anspruch 1, dadurch gekennzeichnet, dass diese 2,3,6-Tridesoxy-4-C-trifluoracetamido-methyl-3-trifluoracetamido-4-O-trifluoracetyl-L-lyxo-hexopyranosyl-chlorid darstellt.

14. Verfahren zur Herstellung von Zwischenverbindungen gemäss den Ansprüchen 11 und 12, gekennzeichnet durch

(A) Behandlung von 2,3,6-Tridesoxy-4-C-methyl-3-trifluoracetamido-α-L-lyxo-hexopyranosid in wässerfreiem Benzol mit Thionylchlorid und anschliessend mit wässrigem Natriumbicarbonat unter Erhalt von 2,3,4,6-Tetradesoxy-4-C-methylen-3-trifluoracetamido-α-L-threo-hexopyranosid;

(B) katalytische Hydrierung der vorstehend erhaltenen Verbindung unter Erhalt von 2,3,4,6-Tetra-desoxy-4-C-methyl-3-trifluoracetamido-α-L-arabino-hexopyranosid;

(C) milde Säurehydrolyse der unter (A) und (B) erhaltenen Verbindungen unter Erhalt der entsprechenden Hexopyranosen, welche durch Behandlung mit Trifluoressigsäureanhydrid in die entsprechenden 1-O-Trifluoracetate umgewandelt werden;

(D) Behandlung mit trockenem Chlorwasserstoff in wasserfreiem Diethylether unter Erhalt der Zwischenverbindungen gemäss den Ansprüchen 11 und 12.

15. Verfahren zur Herstellung der Zwischenverbindung gemäss Anspruch 13, gekennzeichnet durch

(A) Oxidieren von Verbindung VIII

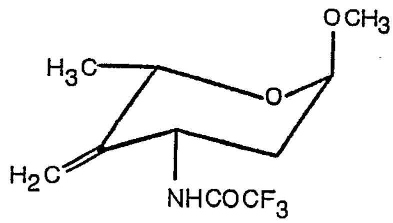

VIII

mit Meta-Chlorperbenzoesäure in 1,2-Dichlorethan, wobei die Verbindung XVI

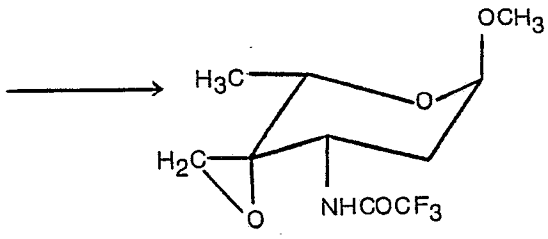

XVI

erhalten wird; oder

(B) Behandlung von Methyl-2,3,6-tridesoxy-3-trifluoracetamido-α-L-threo-hexopyranosid-4-ulose mit Diazomethan, wobei die vorstehend genannte Verbindung XVI erhalten wird,

(C) Azidolyse von Methyl-2,3,6-Tridesoxy-4-C-hydroxy-methyl-4,4'-anhydro-3-trifluoracetamido-α-L-lyxo-hexopyranosid unter Erhalt der entsprechenden Azidoverbindung;

(D) katalytische Hydrierung und anschliessende N-Trifluoracetylierung unter Erhalt der Verbindung XVIII

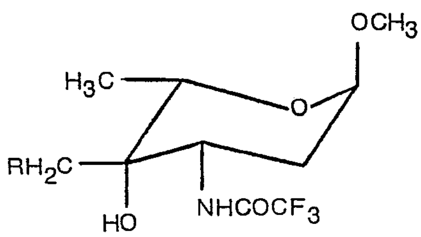

XVIII

worin R = NH—CO—$CF_3$,

(E) welche bei milder Säurehydrolyse die Verbindung XIX ergibt

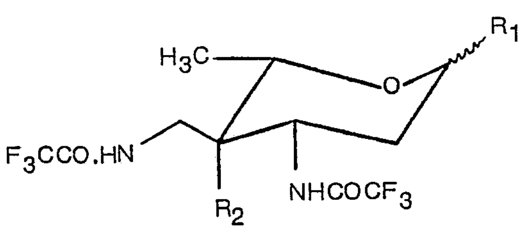

XIX

worin $R_1 = R_2 = OH$;

(F) aufeinanderfolgende O-Trifluoracetylierung und Behandlung mit trockenem Chlorwasserstoff in wasserfreiem Diethylether unter Erhalt des 1-Chlorderivates gemäss Anspruch 13.

16. Pharmazeutische Zubereitung, gekennzeichnet durch eine therapeutisch wirksame Menge einer Verbindung gemäss Anspruch 1 in Kombination mit einem Träger derselben.

**Patentansprüche für den Vertragsstaat: AT**

1. Verfahren zur Herstellung von Anthracyclinglycosiden gemäss Formel I

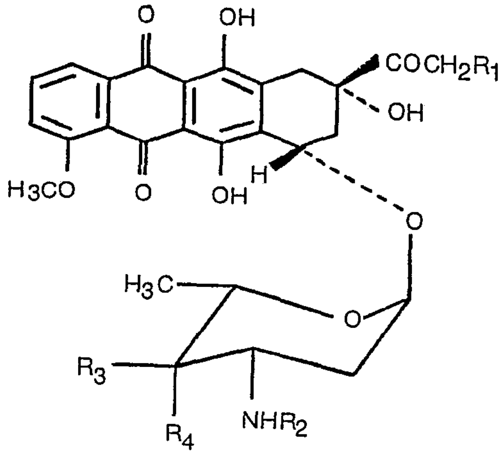

I

worin $R_1$ ein Wasserstoffatom oder eine Hydroxygruppe bedeutet; $R_2$ ein Wasserstoffatom oder eine Acylgruppe darstellt; $R_3$ eine Methyl- oder eine Aminomethylgruppe bedeutet; $R_4$ ein Wasserstoffatom oder eine Hydroxygruppe bedeutet, ausgenommen, dass, wenn $R_3$ eine Methylgruppe bedeutet, $R_4$ nicht eine Hydroxygruppe darstellt, und wenn $R_3$ eine Aminomethylgruppe bedeutet, $R_4$ nicht ein Wasserstoffatom darstellt; $R_3$ und $R_4$ zusammen eine Methylengruppe darstellen können, und deren pharmazeutisch annehmbare Säureadditionssalze, gekennzeichnet durch Kondensieren von Daunomycinon mit

(a) 2,3,4,6-Tetra-desoxy-4-C-methylen-3-trifluoracetamido-L-threo-hexopyranosylchlorid, oder

(b) 2,3,4,6-Tetra-desoxy-4-C-methyl-3-trifluoracetamido-L-arabino-hexopyranosyl-chlorid, oder

(c) 2,3,6-Tridesoxy-4-C-trifluoracetamidomethyl-3-trifluoracetamido-4-O-trifluoracetyl-L-lyxo-hexopyranosyl-chlorid,

um die jeweiligen geschützten Glycoside zu erhalten, und Entfernen der Schutzgruppen unter Erhalt der gewünschten Daunorubicinanaloge.

2. Verfahren nach Anspruch 1, dadurch gekennzeichnet, dass die Daunorubicinanalogen als Hydrochloride isoliert werden.

3. Verfahren zur Herstellung von Anthracyclinglycosiden gemäss Anspruch 1, worin $R_1$ eine Hydroxylgruppe bedeutet, und $R_2$, $R_3$ und $R_4$ die in Anspruch 1 angegebene Bedeutung haben, gekennzeichnet durch C14-Bromierung einer in Anspruch 1 erhaltenen Verbindung und anschliessender Hydrolyse der 14-Bromderivate.

4. Verfahren zur Herstellung von Zwischenverbindungen gemäss Anspruch 1 (a) und (b), gekennzeichnet durch

(A) Behandlung von 2,3,6-Tridesoxy-4-C-methyl-3-trifluor-acetamido-α-L-lyxo-hexopyranosid in wasserfreiem Benzol mit Thionylchlorid und anschliessend mit wässrigem Natriumbicarbonat unter Erhalt von 2,3,4,6-Tetradesoxy-4-C-methylen-3-trifluoracetamido-α-L-threo-hexopyranosid;

(B) katalytische Hydrierung der vorstehend erhaltenen Verbindung unter Erhalt von 2,3,4,8-Tetradesoxy-4-C-methyl-3-trifluoracetamido-α-L-arabino-hexopyranosid;

(C) milde Säurehydrolyse der unter (A) und (B) erhaltenen Verbindungen unter Erhalt der entsprechenden Hexopyranosen, welche durch Behandlung mit Trifluoressigsäureanhydrid in die entsprechenden 1-O-Trifluoracetate umgewandelt werden;

(D) Behandlung mit trockenem Chlorwasserstoff in wasserfreiem Diethylether unter Erhalt der Zwischenverbindungen.

5. Verfahren zur Herstellung einer Zwischenverbindung gemäss Anspruch 1(c), gekennzeichnet durch

(A) Oxidieren von Verbindung VIII

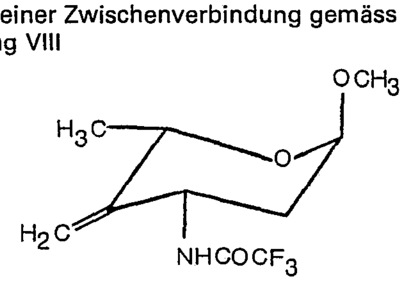

VIII

mit Meta-Chlorperbenzoesäure in 1,2-Dichlorethan, wobei die Verbindung XVI

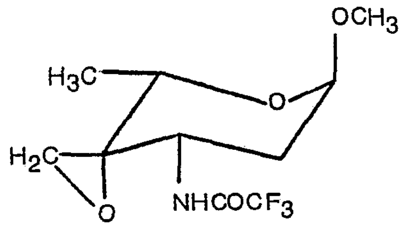

XVI

erhalten wird; oder

(B) Behandlung von Methyl-2,3,6-tridesoxy-3-trifluoracetamido-α-L-threo-hexopyranosid-4-ulose mit Diazomethan, wobei die vorstehend genannte Verbindung XVI erhalten wird,

(C) Azidolyse von Methyl-2,3,6-Tridesoxy-4-C-hydroxy-methyl-4,4'-anhydro-3-trifluoracetamido-α-L-lyxo-hexopyranosid unter Erhalt der entsprechenden Azidoverbindung;

(D) katalytische Hydrierung und anschliessende N-Trifluoracetylierung unter Erhalt der Verbindung XVIII

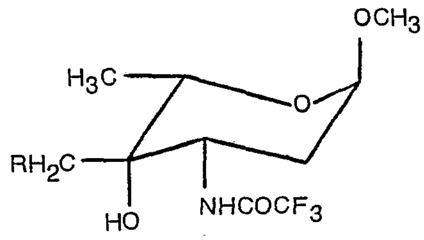

XVIII

worin R = NH—CO—$CF_3$,

(E) welche bei milder Säurehydrolyse die Verbindung XIX ergibt

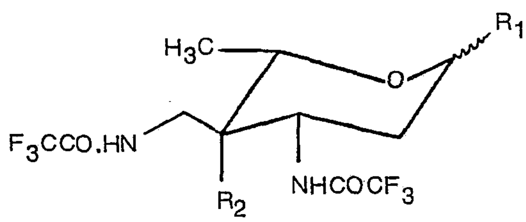

XIX

worin $R_1 = R_2 =$ OH;

(F) aufeinanderfolgende O-Trifluoracetylierung und Behandlung mit trockenem Chlorwasserstoff in wasserfreiem Diethylether unter Erhalt des gewünschten 1-Chlorderivates.

**Revendications pour les Etats contractants: BE CH DE FR GB IT LI LU NL SE**

1. Dérivé glycosidique d'anthracycline de formule générale I

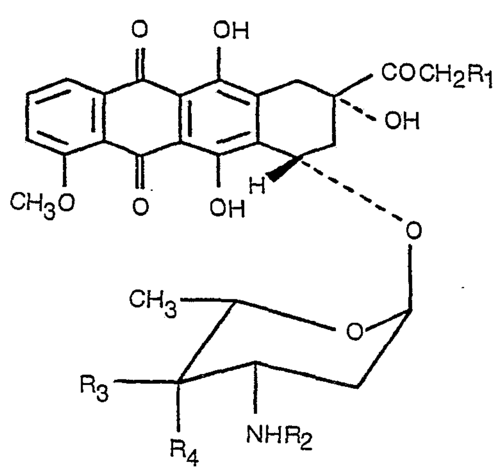

I

dans laquelle $R_1$ représente un atome d'hydrogène ou un groupe hydroxy, $R_2$ représente un atome d'hydrogène ou un groupe acyle, $R_3$ représente un groupe méthyle ou aminométhyle, $R_4$ représente un atome d'hydrogène ou un groupe hydroxy avec la condition que lorsque $R_3$ représente un groupe méthyle, $R_4$ ne représente pas un groupe hydroxy et lorsque $R_3$ représente un groupe aminométhyle, $R_4$ ne repré-

sente pas un atome d'hydrogène; $R_3$ et $R_4$ pouvant représenter ensemble un groupe méthylène, et leurs sels d'addition acides acceptables pharmaceutiquement.

2. Composé selon la revendication 1 qui est le chlorhydrate de 4'-désoxy-4'-C-méthylène-daunorubicine.

3. Composé selon la revendication 1 qui est le chlorhydrate de 4'-désoxy-4'-épi-C-méthyl-daunorubicine.

4. Composé selon la revendication 1 qui est le chlorhydrate de 4'-désoxy-4'-épi-C-méthyl-doxorubicine.

5. Composé selon la revendication 1 qui est le chlorhydrate de 4'-C-amino-méthyl-daunorubicine.

6. Composé selon la revendication 1 qui est le chlorhydrate de 4'-C-amino-méthyl-doxorubicine.

7. Procédé pour la préparation de dérivés glycosidiques d'anthracycline répondant à la formule I de la revendication 1, dans laquelle $R_1$ représente un atome d'hydrogène et $R_2$, $R_3$ et $R_4$ sont définis comme dans cette revendication 1, caractérisé en ce qu'on condense la daunomycinone avec:

a) le chlorure de 2,3,4,6-tétra-désoxy-4-C-méthylène-3-trifluoroacétamido-L-thréo-hexo-pyranosyle, ou

b) le chlorure de 2,3,4,6-tétra-désoxy-4-C-méthyl-3-trifluoroacétamido-L-arabino-hexopyranosyle, ou

c) le chlorure de 2,3,6-tridésoxy-4-C-trifluoroacétamido-méthyl-3-trifluoroacétamido-4-O-trifluoro-acétyl-L-lyxo-hexopyranosyle,

pour obtenir les dérivés glycosidiques protégés respectifs, et on élimine les groupes protecteurs pour obtenir les analogues de daunorubicine désirés.

8. Procédé selon la revendication 7 caractérisé en ce qu'on isole les analogues de daunorubicine sous forme de chlorhydrates.

9. Procédé selon la revendication 7 caractérisé en ce que la condensation de la daunomycinone avec les composés a) à c) selon la revendication 7 est effectuée en présence de trifluorométhanesulfonate d'argent comme catalyseur.

10. Procédé pour la préparation de dérivés glycosidiques d'anthracycline répondant à la formule I de la revendication 1 dans laquelle $R_1$ représente un groupe hydroxyle et $R_2$, $R_3$ et $R_4$ sont tels que définis dans cette revendication 1, caractérisé en ce que la bromation en C-14 d'un composé obtenu selon les revendications 7 à 9 est suivie d'une hydrolyse des dérivés 14-bromo.

11. Composé intermédiaire pour la préparation de dérivés glycosidiques d'anthracycline selon la revendication 1 qui est le chlorure de 2,3,4,6-tétra-désoxy-4-C-méthylène-3-trifluoroacétamido-L-thréo-hexopyranosyle.

12. Composé intermédiaire pour la préparation de dérivés glycosidiques d'anthracycline selon la revendication 1 qui est le chlorure de 2,3,4,6-tétra-désoxy-4-C-méthyl-3-trifluoroacétamido-L-arabino-hexo-pyranosyle.

13. Composé intermédiaire pour la préparation de dérivés glycosidiques d'anthracycline selon la revendication 1 qui est le chlorure de 2,3,6-tridésoxy-4-C-trifluoroacétamidométhyl-3-trifluoroacétamido-4-O-trifluoroacétyl-L-lyxo-hexopyranosyle.

14. Procédé pour la préparation de composés intermédiaires des revendications 11 et 12 caractérisé par:

A) le traitement de 2,3,6-tridésoxy-4-C-méthyl-3-trifluoroacétamido-α-L-lyxo-hexopyranoside dans du benzène anhydre avec du chlorure de thionyle suivi de bicarbonate de sodium aqueux pour obtenir 2,3,4,6-tétradésoxy-4-C-méthylène-3-trifluoroacétamido-α-L-thréo-hexopyranoside;

B) l'hydrogénation catalytique du composé obtenu ci-dessus pour parvenir à 2,3,4,6-tétradésoxy-4-C-méthyl-3-trifluoroacétamido-α-L-arabino-hexopyranoside;

C) l'hydrolyse acide douce des composés obtenus en a) et b) pour obtenir les hexopyranoses correspondants qui sont transformés par traitement avec de l'anhydride trifluoroacétique en 1-O-trifluoroacétates correspondants;

D) le traitement avec de l'acide chlorhydrique sec dans de de l'éther diéthylique anhydre pour obtenir les composés intermédiaires des revendications 11 et 12.

15. Procédé pour la préparation du composé intermédiaire selon la revendication 13 caractérisé par:

A) l'oxydation du composé VIII

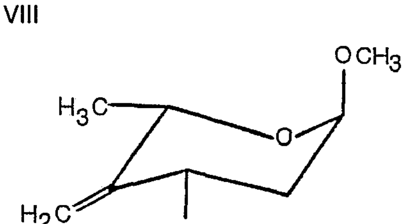

VIII

avec de l'acide méta-chloroperbenzoïque en 1,2-dichloroéthane, de sorte que le composé XVI

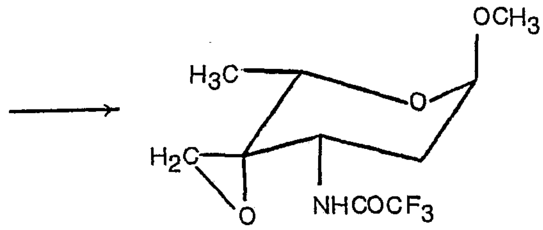

XVI

est obtenu, ou

B) le traitement de méthyl 2,3,6-tridésoxy-3-trifluoroacétamido-α-L-thréo-hexopyranoside-4-ulose avec du diazométhane, de sorte que le composé XVI mentionné ci-dessus est obtenu,

C) l'azidolyse de méthyl-2,3,6-tridésoxy-4-C-hydroxy-méthyl-4,4'-anhydro-3-trifluoroacétamido-α-L-lyxo-hexopyranoside pour donner le composé azido respectif,

D) l'hydrogénation catalytique suivie d'une N-trifluoroacétylation pour donner le composé XVIII

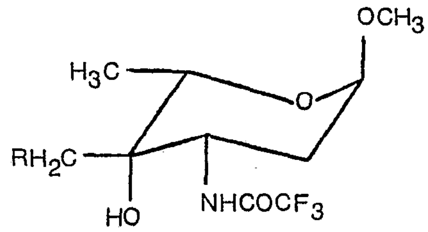

XVIII

dans lequel R = NH—CO—$CF_3$,

E) qui procure par une hydrolyse acide douce le composé XIX

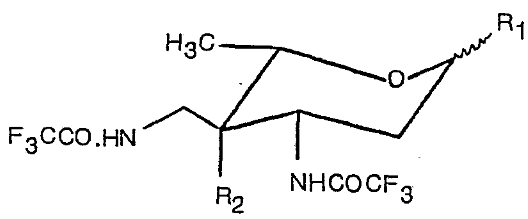

XIX

dans lequel $R_1$ = $R_2$ = OH,

F) une O-trifluoroacétylation séquentielle et le traitement avec de l'acide chlorhydrique sec dans de l'éther diéthylique anhydre pour obtenir le dérivé 1-chloro de la revendication 13.

16. Composition pharmaceutique comprenant une quantité efficace au point de vue thérapeutique d'un composé selon la revendication 1 en combinaison avec un véhicule de ce composé.

**Revendications pour l'Etat contractant: AT**

1. Procédé pour la préparation de dérivés glycosidiques d'anthracycline répondant à la formule I

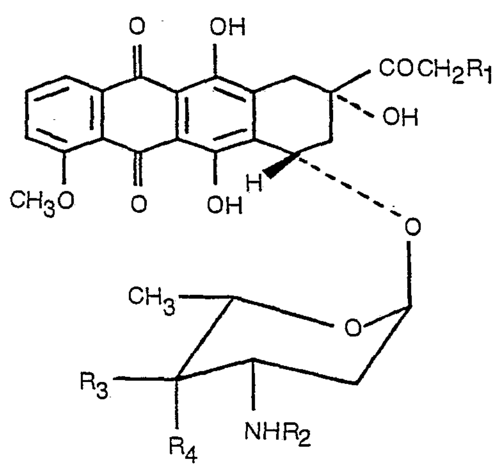

I

dans laquelle $R_1$ représente un atome d'hydrogène ou un groupe hydroxy; $R_2$ représente un atome d'hydrogène ou un groupe acyle; $R_3$ représente un groupe méthyle ou aminométhyle; $R_4$ représente un

atome d'hydrogène ou un groupe hydroxy avec la condition que lorsque $R_3$ représente un groupe méthyle, $R_4$ ne représente pas un groupe hydroxy et lorsque $R_3$ représente un groupe aminométhyle, $R_4$ ne représente pas un atome d'hydrogène; $R_3$ et $R_4$ pouvant représenter ensemble un groupe méthylène, et leurs sels d'addition acides acceptables pharmaceutiquement, caractérisé en ce qu'on condense de la daunomycinone avec

(a) le chlorure de 2,3,4,6-tétra-désoxy-4-C-méthylène-3-trifluoroacétamido-L-thréo-hexo-pyranosyle, ou

(b) le chlorure de 2,3,4,6-tétra-désoxy-4-C-méthyl-3-trifluoroacétamido-L-arabino-hexopyranosyle, ou

(c) chlorure de 2,3,6-tridésoxy-4-C-trifluoroacétamidométhyl-3-trifluoroacétamido-4-O-trifluoroacétyl-L-lyxo-hexopyranosyle

pour obtenir les dérivés glycosidiques protégés respectifs, et on élimine les groupes protecteurs pour obtenir les analogues de daunorubicine désirés.

2. Procédé selon la revendication 1 caractérisé en ce qu'on isole les analogues de daunorubicine sous forme de chlorhydrates.

3. Procédé pour la préparation de dérivés glycosidiques d'anthracycline selon la revendication 1 dans lesquels $R_1$ représente un groupe hydroxyle et $R_2$, $R_3$ et $R_4$ sont tels que définis dans cette revendication 1, caractérisé par une bromation en C14 d'un composé tel qu'obtenu à la revendication 1, suivie d'un hydrolyse des dérivés 14-bromo.

4. Procédé pour la préparation de composés intermédiaires selon la revendication 1 (a) et 1 (b), caractérisé par

A) le traitement de 2,3,6-tridésoxy-4-C-méthyl-3-trifluoroacétamido-α-L-lyxo-hexopyranoside dans du benzène anhydre avec du chlorure de thionyle suivi de bicarbonate de sodium aqueux pour obtenir 2,3,4,6-tétradésoxy-4-C-méthylène-3-trifluoroacétamido-α-L-thréo-hexopyranoside;

B) l'hydrogénation catalytique du composé obtenu ci-dessus pour parvenir à 2,3,4,6-tétradésoxy-4-C-méthyl-3-trifluoroacétamido-α-L-arabino-hexopyranoside;

C) l'hydrolyse acide douce des composés obtenus en A) et B) pour obtenir les hexopyranoses correspondants qui sont transformés par traitement avec de l'anhydride trifluoroacétique en 1-O-trifluoroacétates correspondants;

D) le traitement avec de l'acide chlorhydrique sec dans de l'éther diéthylique anhydre pour obtenir les composés intermédiaires désirés.

5. Procédé pour la préparation du composé intermédiaire selon la revendication 1 (c) caractérisé par

A) l'oxydation du composé VIII

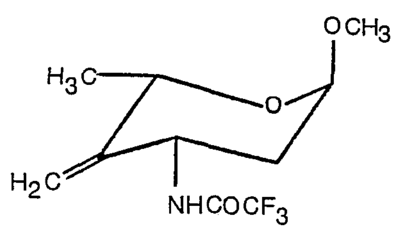

VIII

avec de l'acide méta-chloroperbenzoïque en 1,2-dichloroéthane, de sorte que le composé XVI

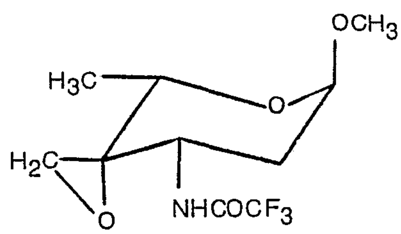

XVI

est obtenu, ou

B) le traitement de méthyl 2,3,6-tridésoxy-3-trifluoroacétamido-α-L-thréo-hexopyranoside-4-ulose avec du diazométhane, de sorte que le composé XVI mentionné ci-dessus est obtenu,

C) l'azidolyse de méthyl-2,3,6-tridésoxy-4-C-hydroxy-méthyl-4,4'-anhydro-3-trifluoroacétamido-α-L-lyxo-hexopyranoside pour donner le composé azido respectif,

D) l'hydrogénation catalytique suivie d'une N-trifluoroacétylation pour donner le composé XVIII

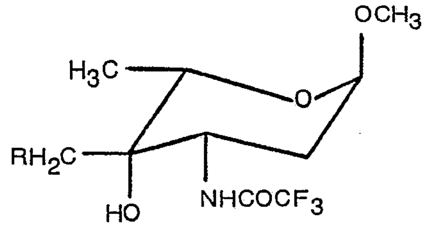

XVIII

dans lequel $R = NN—CO—CF_3$,

E) qui procure par une hydrolyse acide douce le composé XIX

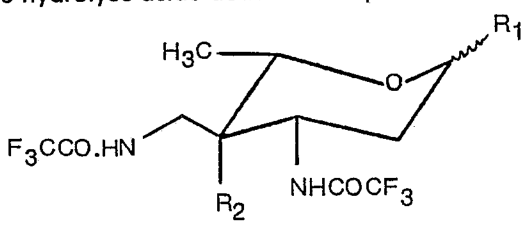

XIX

dans lequel $R_1 = R_2 = OH$,

F) une O-trifluoroacétylation séquentielle et le traitement avec de l'acide chlorhydrique sec dans de l'éther diéthylique anhydre pour obtenir le dérivé 1-chloro désiré.